# EUROPEAN PATENT APPLICATION

(11) **EP 4 385 501 A1**
(43) Date of publication of application: **19.06.2024**
(21) Application number: 23216893.0
(22) Date of filing: 14.12.2023
(51) Int. Cl.: A61K 9/20, A61K 31/4439, A61K 31/522, A61K 31/7048, A61K 31/155

(54) **A PHARMACEUTICAL FORMULATION COMPRISING LINAGLIPTIN, PIOGLITAZONE AND A SGLT-2 INHIBITOR**

(30) Priority: 15.12.2022 TR 202219413
(71) Applicant: SANOVEL ILAC SANAYI VE TICARET A.S., Istanbul 34460 (TR)
(72) Inventor: ARI, Busra, Istanbul (TR); PEHLIVAN AKALIN, Nur, Istanbul (TR); SUNEL, Fatih, Istanbul (TR); MUTLU, Onur, Istanbul (TR)
(74) Representative: Sevinç, Erkan

(57) **Abstract**

The present invention relates to the pharmaceutical formulation comprising linagliptin or salts thereof, pioglitazone or salts thereof and a SGLT-2 inhibitor.

## Description

### Field of the Invention

The present invention relates to the pharmaceutical formulation comprising linagliptin or salts thereof, pioglitazone or salts thereof and a SGLT-2 inhibitor.

### Background of the Invention

Diabetes mellitus is a group of disorders of carbohydrate metabolism in which the action of insulin is diminished or absent through altered secretion, decreased insulin activity or a combination of both factors.

Linagliptin is used for type 2 or non-insulin dependent diabetes. It is a selective, orally administered, xanthine based dipeptidyl peptidase-4 (DPP-4) inhibitor used as an adjunct to diet and exercise to improve glycemic control. DPP-4 inhibitors work by blocking the action of DPP-4, an enzyme which destroys the hormone incretin. There are two types of incretin hormones found in the body, called glucagon-like peptide-1 (GLP-1) and glucose-dependent insulinotropic peptide (GIP). These hormones are naturally produced by the body in response to food intake. Their function is to help the body produce more insulin only when it is needed and reduce the amount of glucose being produced by the liver when it is not needed. Linagliptin works by binding to DPP-4 and preventing it from breaking down the GLP-1 and GIP. This increases the levels of these hormones in the body and so increases their effect on controlling blood sugar.

The chemical name of linagliptin is 8-[(3R)-3-aminopiperidin-1-yl]-7-but-2-yn-1-yl)-3-methyl-1-[(4-methylquinazolin-2-yl)methyl]-3,7-dihydro-1H-purine-2,6-dione and its chemical structure is shown in the Formula I.

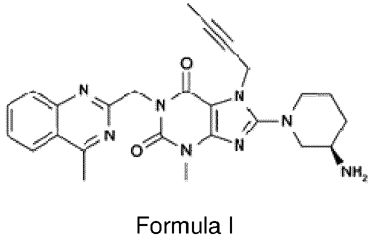

Pioglitazone is an orally-administered antidiabetics. Pioglitazone belongs to the class of thiazolinediones which act primarily by decreasing insulin resistance. It is not practically soluble in water.

On the other hand, the chemical designation of pioglitazone hydrochloride is (+/-)-5-[[4-[2-(5-ethyl-2-pyridinyl)ethoxy]phenyl]methyl]-2,4-thiazolidinedione hydrochloride, with the following chemical structure of Formula 2.

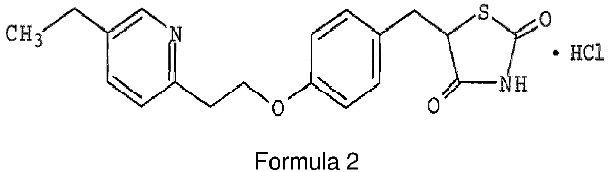

Pioglitazone is disclosed in the patent EP0193256.

The combination comprises linagliptin and pioglitazone formulations have been disclosed by Boehringer Pharmaceutical.

It is known for treating a type 2 diabetes in a mammalian patient who has previously been treated with one or more oral anti-diabetic agents and/or one or more injectable anti-diabetic agents (e.g., insulin). Sometimes these are considered insufficient.

Limited improvement in glycemic control is observed with long-term treatment. In addition, even in patients within the intensive treatment glycemic control can deteriorated significantly over time by deterioration of β-cell function.

The SGLT2 inhibitor is a class of medications that modulate sodium-glucose transport proteins in the nephron and is used in the treatment of type II diabetes mellitus. Apart from blood sugar control, gliflozins have been shown to provide significant cardiovascular benefits in patients with type II diabetes. SGLT2 inhibitor is administered at a dose from about 0.5 to about 200 mg/day, and insulin or other oral anti-diabetic agents are also administered at a dose as prescribed by a doctor.

Therefore, there is thus still a need for methods, medicaments and pharmaceutical combinations with a good efficacy with regard to glycemic control, with regard to disease-modifying properties and with regard to reduction of cardiovascular morbidity and mortality while at the same time showing an improved safety profile.

In this invention, combining more than two molecules in one dosage form increases the patient's compliance. The combination brings additional advantages to the relevant prior art. The term "combination" means that when drugs are administered together, a combined action is obtained which is higher than the individual actions of the respective drugs when they are used separately. On the other hand, using a lower dose of each drug to be combined according to the present invention will reduce the total dosage. These are advantageous in terms of patients to be treated.

### Detailed Description of the Invention

The main object of the present invention is to obtain a stable combination formulation with synergistic effect for use in treatment of a type 2 diabetes in a mammalian.

Another object of the present invention is to provide a formulation comprising linagliptin or salts thereof, pioglitazone or salts thereof and a SGLT-2 inhibitor.

Another object of the present invention is to provide a formulation comprising linagliptin or salts thereof, pioglitazone or salts thereof and a SGLT-2 inhibitor having the desired dissolution profile, homogeneity, high stability, and flowability.

According to one embodiment of the present invention, the pharmaceutical formulation comprises;
- Linagliptin or salts thereof,
- Pioglitazone or salts thereof,
- A SGLT-2 inhibitor.

SGLT2 inhibitor is selected from the group comprising canagliflozin, dapagliflozin, empagliflozin, ertugliflozin, ipragliflozin, luseogliflozin, remogliflozin etabonate, tofogliflozin.

According to one embodiment of the present invention, the SGLT2 inhibitor is dapagliflozin.

According to one embodiment of the present invention, the SGLT2 inhibitor is empagliflozin.

According to one embodiment of the present invention, the SGLT2 inhibitor is canagliflozin.

According to one embodiment of the present invention, the SGLT2 inhibitor is ertugliflozin.

According to one embodiment of the present invention, the SGLT2 inhibitor is ipragliflozin.

According to one embodiment of the present invention, the SGLT2 inhibitor is tofogliflozin.

According to one embodiment of the present invention, the SGLT2 inhibitor is luseogliflozin.

According to one embodiment of the present invention, the SGLT2 inhibitor is remogliflozin etabonate.

According to one embodiment of the present invention, the amount of linagliptin or salts thereof is between 1.0% and 10.0% by weight in the total formulation.

According to one embodiment of the present invention, the amount of pioglitazone or salts thereof is between 0.1% and 10.0% by weight in the total formulation.

According to one embodiment of the present invention, the amount of a SGLT2 inhibitor is between 0.5% and 15.0% by weight in the total formulation.

According to one embodiment of the present invention, the pharmaceutical formulation comprises linagliptin is present in an amount of 5mg.

According to one embodiment of the present invention, the pharmaceutical formulation comprises pioglitazone is present in an amount of between 15 mg and 30 mg.

According to one embodiment of the present invention, the pharmaceutical formulation comprises a SGLT2 inhibitor is present in an amount of between 5 mg and 20 mg.

In general terms, excipients provided in a formulation may positively or negatively influence the physicochemical and pharmacokinetic properties, e.g. the solubility, absorption, bioavailability of active agents. For this reason, the excipients which accompany active agents have to be selected in a careful and conscious manner while a formulation is developed. The formulations should have no physicochemical incompatibility between the active agents and the excipients.

According to this embodiment of the present invention, the formulation further comprises at least one pharmaceutically acceptable excipient which is selected from the group comprising fillers, binders, disintegrants, lubricants, glidants or mixtures thereof.

Suitable fillers are selected from the group comprising ammonium alginate, calcium carbonate, calcium phosphate, calcium phosphate dehydrate, neutral pellets, calcium sulfate, cellulose acetate, compressible sugar, erythritol, ethylcellulose, fructose, glyceryl palmitostearate, lactose, mannitol, lactose monohydrate, magnesium carbonate, magnesium oxide, maltodextrin, maltose, medium chain triglycerides, microcrystalline cellulose, polydextrose, polymethacrylates, sodium alginate, sodium chloride, sorbitol, starch, sucrose, sugar spheres, sulfobutylether beta-cyclodextrin, talc, tragacanth, trehalose, polysorbate 80, xylitol or mixtures thereof.

According to one embodiment of the present invention, the filler is microcrystalline cellulose or lactose or lactose monohydrate or mannitol or mixtures thereof.

According to one embodiment of the present invention, the amount of the filler is between 10.0% and 70.0 by weight in the total formulation. Preferably, the amount of the filler is between 25.0% and 60.0 by weight in the total formulation. The amount of the filler provides the desired content uniformity and flowability.

Suitable binders are selected from the group comprising polyvinylpyrrolidone, carboxymethylcellulose sodium, cellulose acetate phthalate, starch, corn starch, pregelatinized starch, ethylcellulose, glyceryl behenate, hydrogenated vegetable oil type I, hydroxyethyl cellulose, hydroxyethylmethyl cellulose, hydroxypropyl cellulose, hydroxypropyl starch, hydroxypropyl methyl cellulose, polyoxyethilene-alkyl ethers or mixtures thereof.

According to one embodiment of the present invention, the binder is polyvinylpyrrolidone or corn starch or HPMC or mixtures thereof. The binders help to provide the desired stability.

According to one embodiment of the present invention, the amount of the binder is between 0.5% and 10.0 by weight in the total formulation.

Suitable disintegrants are selected from the group comprising crospovidone, croscarmellose sodium, low-substituted hydroxypropyl cellulose, calcium carboxymethyl cellulose, carboxymethyl cellulose, sodium alginate, sodium starch glycolate, sodium glycine carbonate or mixtures thereof.

According to one embodiment of the present invention, the disintegrant is crospovidone or croscarmellose sodium or mixtures thereof.

According to one embodiment of the present invention, the amount of disintegrant is between 1.0% and 15.0% by weight in the total formulation. The ratio helps to provide the desired dissolution profile.

Suitable lubricants are selected from the group comprising magnesium stearate, calcium stearate, zinc stearate, talc, sodium chlorate, magnesium lauryl sulfate, sodium oleate, sodium acetate, sodium benzoate, polyethylene glycol, sodium stearyl fumarate, sodium lauryl sulphate or mixtures thereof.

According to one embodiment of the present invention, the lubricant is magnesium stearate or talc or sodium stearyl fumarate or mixtures thereof.

Suitable glidants are selected from the group comprising aluminum silicate, colloidal silicon dioxide, colloidal silica, calcium silicate, magnesium silicate, magnesium oxide, starch or mixtures thereof.

According to one embodiment of the present invention, the pharmaceutical formulation is in the form of tablets, capsules, orally disintegrating tablets, effervescent compositions, hard or soft gelatin capsules, coated bead systems, granules, microspheres.

According to one embodiment of the present invention, the pharmaceutical formulation is in the form of tablet or capsule.

According to one embodiment of the present invention, tablets are selected from the group comprising film-coated tablets, bilayer tablets, inlay tablets, orally disintegrating tablets, multilayer tablets, mini tablets, buccal tablets, sublingual tablets, effervescent tablets, immediate release tablets, modified release tablets, gastric disintegrating tablets.

According to one embodiment of the present invention, the pharmaceutical formulation is formulated as film coated tablet or bilayer tablet or multilayer tablet or modified release tablet.

According to another embodiment of the present invention, the pharmaceutical formulation is formulated in capsule form which is selected from the group comprising hard capsule, soft capsule, and enteric capsule. The capsule comprises at least one type of particle, for example; mini-capsules, mini-tablets, pellets, cores, agglomerates, granules, powders, liposomes, spherical or mixtures thereof.

The pharmaceutical formulation of the present invention can be prepared, using standard techniques and manufacturing processes well known in the art, such as direct compression, wet or dry granulation, hot melt granulation, hot melt extrusion, fluidized bed granulation, extrusion/spheronization, slugging, spray drying and solvent evaporation.

According to another embodiment of the present invention, the formulation is prepared, using wet or dry granulation.

Solvent is used in wet granulation. Suitable solvents are selected from the group comprising dichloromethane, 0.1N HCl, water, methanol, ethanol, isopropyl alcohol, benzyl alcohol, propylene glycol, polyethylene glycol, glycerine, cyclomethicone, glycerine triacetate, diethylene glycol monoethyl ether, propylene carbonate or mixtures thereof. Preferably, solvent is water or ethanol or mixtures thereof.

A process for preparing a pharmaceutical formulation comprising linagliptin, pioglitazone and a SGLT-2 inhibitor comprising the following steps:
a) Mixing linagliptin, pioglitazone and a SGLT-2 inhibitor, at least one filler, at least one binder and at least one disintegrant,
b) Granulating the powder mixture with a solvent,
c) Sieving the wet granules and then drying,
d) Sieving the dried granules and adding at least one lubricant,
e) Obtaining the total mixture into the desired shape (tablet or granule or powder),
f) Filling the mixture into a capsule or compressing the mixture at a tablet.

A process for preparing a pharmaceutical formulation comprising linagliptin, pioglitazone and a SGLT-2 inhibitor comprising the following steps:
a) Mixing linagliptin, pioglitazone and a SGLT-2 inhibitor, at least one filler, at least one binder and at least one disintegrant,
b) Granulating the powder mixture with a solvent,
c) Sieving the wet granules and then drying,
d) Sieving the dried granules and adding at least one lubricant,
e) Obtaining the total mixture into the desired shape (tablet or granule or powder),
f) Filling the mixture into a capsule or compressing the mixture at a tablet.

### Example 1: A tablet or a capsule

| **Ingredients** | **Amount (% by weight of the total formulation)** |
|---|---|
| Linagliptin | 1.0 - 10.0 |
| Pioglitazone | 0.1 - 10.0 |
| Empagliflozin | 0.5 - 15.0 |
| Filler | 10.0 - 70.0 |
| Binder | 0.5 - 10.0 |
| Disintegrant | 1.0 - 15.0 |
| **TOTAL** | **100** |

### Example 2: A tablet or a capsule

| **Ingredients** | **Amount (% by weight of the total formulation)** |
|---|---|
| Linagliptin | 1.0 - 10.0 |
| Pioglitazone | 0.1 - 10.0 |
| Dapagliflozin | 0.5 - 15.0 |
| Filler | 10.0 - 70.0 |
| Binder | 0.5 - 10.0 |
| Disintegrant | 1.0 - 15.0 |
| **TOTAL** | **100** |

### Example 3: A pharmaceutical formulation

| **Ingredients** | **Amount (% by weight of the total formulation)** |
|---|---|
| Linagliptin | 1.0 - 10.0 |
| Pioglitazone | 0.1 - 10.0 |
| Dapagliflozin or empagliflozin | 0.5 - 15.0 |
| Microcrystalline cellulose or lactose or lactose monohydrate or mannitol as filler | 10.0 - 70.0 |
| Hypromellose and polyvinylpyrrolidone as binder | 0.5-10.0 |
| crospovidone or croscarmellose sodium as disintegrant | 1.0 - 15.0 |
| Other excipients | 1.0 - 20.0 |
| **TOTAL** | **100** |

## Claims

1. A pharmaceutical formulation comprising;
- Linagliptin or salts thereof,
- Pioglitazone or salts thereof,
- A SGLT-2 inhibitor.

2. The pharmaceutical formulation according to claim 1, wherein SGLT2 inhibitor is selected from the group comprising canagliflozin, dapagliflozin, empagliflozin, ertugliflozin, ipragliflozin, luseogliflozin, remogliflozin etabonate, tofogliflozin.

3. The pharmaceutical formulation according to claim 2, wherein the SGLT2 inhibitor is dapagliflozin.

4. The pharmaceutical formulation according to claim 2, wherein the SGLT2 inhibitor is empagliflozin.

5. The pharmaceutical formulation according to claim 1, wherein the amount of linagliptin or salts thereof is between 1.0% and 10.0% by weight in the total formulation.

6. The pharmaceutical formulation according to claim 1, wherein the amount of pioglitazone or salts thereof is between 0.1% and 10.0% by weight in the total formulation.

7. The pharmaceutical formulation according to claim 1, wherein the amount of a SGLT2 inhibitor is between 0.5% and 15.0% by weight in the total formulation.

8. The pharmaceutical formulation according to claim 1, wherein the formulation further comprises at least one pharmaceutically acceptable excipient which is selected from the group comprising fillers, binders, disintegrants, lubricants, glidants or mixtures thereof.

9. The pharmaceutical formulation according to claim 8, wherein fillers are selected from the group comprising ammonium alginate, calcium carbonate, calcium phosphate, calcium phosphate dehydrate, neutral pellets, calcium sulfate, cellulose acetate, compressible sugar, erythritol, ethylcellulose, fructose, glyceryl palmitostearate, lactose, mannitol, lactose monohydrate, magnesium carbonate, magnesium oxide, maltodextrin, maltose, medium chain triglycerides, microcrystalline cellulose, polydextrose, polymethacrylates, sodium alginate, sodium chloride, sorbitol, starch, sucrose, sugar spheres, sulfobutylether beta-cyclodextrin, talc, tragacanth, trehalose, polysorbate 80, xylitol or mixtures thereof.

10. The pharmaceutical formulation according to claim 9, wherein the filler is microcrystalline cellulose or lactose or lactose monohydrate or mannitol or mixtures thereof.

11. The pharmaceutical formulation according to claim 8, wherein binders are selected from the group comprising polyvinylpyrrolidone, carboxymethylcellulose sodium, cellulose acetate phthalate, starch, corn starch, pregelatinized starch, ethylcellulose, glyceryl behenate, hydrogenated vegetable oil type I, hydroxyethyl cellulose, hydroxyethylmethyl cellulose, hydroxypropyl cellulose, hydroxypropyl starch, hydroxypropyl methyl cellulose, polyoxyethilene-alkyl ethers or mixtures thereof.

12. The pharmaceutical formulation according to claim 11, wherein the binder is polyvinylpyrrolidone or corn starch or HPMC or mixtures thereof.

13. The pharmaceutical formulation according to claim 8, wherein disintegrants are selected from the group comprising crospovidone, croscarmellose sodium, low-substituted hydroxypropyl cellulose, calcium carboxymethyl cellulose, carboxymethyl cellulose, sodium alginate, sodium starch glycolate, sodium glycine carbonate or mixtures thereof.

14. The pharmaceutical formulation according to claim 13, wherein the disintegrant is crospovidone or croscarmellose sodium or mixtures thereof.

15. The pharmaceutical formulation according to claim 8, wherein lubricants are selected from the group comprising magnesium stearate, calcium stearate, zinc stearate, talc, sodium chlorate, magnesium lauryl sulfate, sodium oleate, sodium acetate, sodium benzoate, polyethylene glycol, sodium stearyl fumarate, sodium lauryl sulphate or mixtures thereof.
